# EUROPÄISCHE PATENTSCHRIFT

(11) **EP 1 608 388 B1**
(45) Veröffentlichungstag und Bekanntmachung des Hinweises auf die Patenterteilung: **10.10.2007**
(21) Anmeldenummer: 04724580.8
(22) Anmeldetag: 31.03.2004
(51) Int. Cl.: A61K 36/25, A61P 11/00

(54) **VERFAHREN ZUR HERSTELLUNG EINES EXTRAKTES AUS EFEUBLÄTTERN**
METHOD FOR THE PRODUCTION OF AN EXTRACT OF IVY LEAVES
PROCEDE POUR PRODUIRE UN EXTRAIT A PARTIR DE FEUILLES DE LIERRE

(30) Priorität: 02.04.2003 DE 10315931
(43) Veröffentlichungstag der Anmeldung: 28.12.2005
(73) Patentinhaber: Engelhard Arzneimittel GmbH & Co. KG, 61138 Niederdorfelden (DE)
(72) Erfinder: RUNKEL, Frank, 35418 Buseck (DE); ENGELHARD, Karl-Michael, 61381 Friedrichsdorf (DE); ENGELHARD, Georg, Maximilian, 61476 Kronberg (DE)
(74) Vertreter: Weller, Wolfgang
(86) Internationale Anmeldenummer: PCT/EP2004/003420
(87) Internationale Veröffentlichungsnummer: WO 2004/087183

(56) Entgegenhaltungen:
- DE-A- 2 707 675
- US-A- 4 684 522
- DATABASE CHEMABS [Online] CHEMICAL ABSTRACTS SERVICE, COLUMBUS, OHIO, US; 1949, COELHO, FERNANDO PINTO: "The determination of the structure of the tetracyclic triterpene alcohol, basseol. II. The dehydrogenation of basseol and of bassenol-triterpenes of the .beta.-amyrenol group" XP002287962 gefunden im STN Database accession no. 45:29613 & REV. FACULDADE CIENC. UNIV., COIMBRA , 18, 71-140, 1949,
- ELIAS R ET AL: "Influence of the drying's process and the alcoholic degree on the extraction of hederasaponin C and [alpha]-hederin from the leaves of Hedera helix L." JOURNAL DE PHARMACIE DE BELGIQUE 1991 FRANCE, Bd. 46, Nr. 3, 1991, Seiten 177-181, XP009033524 ISSN: 0047-2166
- DATABASE SCISEARCH [Online] 2002, HECKER M ET AL: "Treatment of Chronic Bronchitis with an ivy leaf special extract - Multicenter post-marketing surveillance study in 1350 patients" XP002287963 gefunden im STN Database accession no. 2002:437361 & FORSCHENDE KOMPLEMENTARMEDIZIN UND KLASSISCHE NATURHEILKUNDE, (APR 2002) VOL. 9, NO. 2, PP. 77-84. PUBLISHER: KARGER, ALLSCHWILERSTRASSE 10, CH-4009 BASEL, SWITZERLAND. ISSN: 1424-7364., 2002,
- DATABASE BIOSIS [Online] BIOSCIENCES INFORMATION SERVICE, PHILADELPHIA, PA, US; 1997, TRUTE ANDREAS ET AL: "In vitro antispasmodic compounds of the dry extract obtained from Hedera helix" XP002287964 Database accession no. PREV199799569819 & PLANTA MEDICA, Bd. 63, Nr. 2, 1997, Seiten 125-129, ISSN: 0032-0943

## Beschreibung

Die vorliegende Erfindung betrifft ein Verfahren zur Herstellung eines Extraktes aus Efeublättern unter Verwendung eines Extraktionsmittels, wobei der Trockenextrakt α-Hederin aufweist.

Extrakte aus Efeublättern werden heutzutage erfolgreich vor allem zur Therapie von Atemwegserkrankungen eingesetzt, da der Extrakt spasmolytische, expektorierende und antiobstruktive Wirkungen zeigt. Diese Wirkungen sind insbesondere auf die therapeutisch wichtigen Inhaltsstoffe der Efeublätterextrakte zurückzuführen, die der Klasse der Triterpensaponine angehören. Hauptsaponin ist dabei das bisdesmosidische Hederacosid C und das daraus durch Esterhydrolyse entstehende α-Hederin. Als weiteres Saponin konnte Hederagenin nachgewiesen werden.

Da Extrakte aus Efeublättern durch verschiedene Verfahren gewonnen werden können, weisen diese Extrakte oftmals unterschiedliche Wirkungsgrade auf. Dies rührt daher, dass der Anteil der Inhaltsstoffe von der jeweiligen Methode der Extraktherstellung abhängt.

Die Anmelderin hat aufgrund von Studien festgestellt, dass insbesondere α-Hederin zur Bronchospasmolyse beiträgt. Es wurde festgestellt, dass α-Hederin den Internalisierungsprozess von β-adrenergen Rezeptoren in spezifischer Weise hemmt. Nach Bindung eines Liganden an die β-adrenergen Rezeptoren wird zunächst das Adenylatcyclase-System aktiviert, über welches als Folgereaktion bspw. die glatte Muskulatur des Bronchialsystems zur Erschlaffung gebracht wird. Einem Überschießen des Effektes wird u.a. durch Herunterregulierung der membranständigen Rezeptoren durch deren Internalisierung in die Zelle entgegengewirkt.

In diesem Zusammenhang konnte gezeigt werden, dass α-Hederin den β-adrenergen Rezeptor nicht direkt angreift, sondern vielmehr die Rezeptor-Ligand-Internalisierung in die Zelle hemmt. Wird die Internalisierung der Rezeptor-Ligand-Komplexe gehemmt, findet eine fortgesetzte Aktivierung des Adenylatcyclase-Systems statt, wodurch in den Bronchien die Erschlaffung der glatten Muskulatur forciert wird (Spasmolyse).

Die Substanzen Hederacosid-C oder Hederagenin hingegen konnten den Vorgang der Internalisierung nicht inhibieren. Demzufolge sind Extrakte mit einem hohen Gehalt an α-Hederin für einen Einsatz als Arzneimittel besonders geeignet.

Verfahren zur Herstellung von Trockenextrakten aus Pflanzenmaterialien sind im Gebiet der Pharmazie und insbesondere der Arzneimittelzubereitungen vielfältig beschrieben.

Ein Verfahren zur Herstellung von Trockenextrakten aus Pflanzenmaterialien ist beispielsweise aus der DE 101 12 168 A1 bekannt. Mit dem dort offenbarten Verfahren soll der Gehalt an lipophilen und hydrophilen Substanzen eingestellt werden können. Hierbei wird das Pflanzenmaterial mindestens zwei Extraktionen mit Lösungsmitteln unterschiedlicher Lipophilie unterzogen und die Extrakte daraus separat gewonnen. Die Extrakte werden getrennt voneinander getrocknet und im gewünschten Verhältnis gemischt. Auf diese Weise kann der Gehalt an lipophilen und hydrophilen Substanzen eingestellt werden. Das Verfahren soll auch zur Gewinnung von Trockenextrakten aus Efeu (*Hedera helix*) geeignet sein.

Nachteilig an diesem Verfahren ist jedoch der Umstand, dass zwei getrennte Extraktionen durchgeführt werden müssen, wodurch das Verfahren insgesamt sehr aufwändig wird.

Ferner ist aus der DE 30 25 223 A1 eine pharmazeutische Zubereitung auf der Grundlage von Efeuextrakten und einem Verfahren zu deren Herstellung bekannt, bei welchem unter Verwendung von Aceton und Methanol ein Efeuextrakt mit einem Hederasaponin C-Gehalt von 60 bzw. 90 % gewonnen wird. Um Hederasaponin C, bzw. Hederacosid C, in α-Hederin umzuwandeln, und um dadurch einen Extrakt mit lediglich α-Hederin zu gewinnen, wird in der genannten Anmeldung der 90%-ige Extrakt mit Natriumhydroxid oder Kaliumhydroxid verseift.

Nachteilig an diesem Verfahren ist jedoch der hohe Aufwand, der durch die weiteren Schritte hervorgerufen wird, die nach der Gewinnung von Hederacosid C notwendig sind, um das Hederacosid, bzw. das Hederasaponin, in α-Hederin umzuwandeln.

Aus G. Wulff "Neuere Entwicklungen auf dem Saponingebiet", Deutsche Apotheker Zeitung, 108 (Nr. 23), 1968, S. 797-808 ist bekannt, dass, falls man frische Efeublätter zerkleinert und über Nacht in Wasser stehen lässt, man anschließend nur noch α-Hederin findet. Dafür werden Enzyme verantwortlich gemacht, die bei frischen Blättern vorhanden sind.

In der pharmazeutischen Industrie werden allerdings getrocknete Drogen eingesetzt, da diese haltbar und besser handhabbar sind. Enzymatische Aktivitäten in solchen getrockneten Drogen sind nicht zu erwarten.

Aufgabe der vorliegenden Erfindung ist es daher, ein Verfahren bereitzustellen, mit welchem mit wenigen Schritten und ohne großen Zeitaufwand ein Extrakt, insbesondere ein Trockenextrakt, gewonnen werden kann, in welchem α-Hederin zumindest stark angereichert vorliegt.

Gemäß dem eingangs genannten Verfahren wird die der Erfindung zu Grunde liegende Aufgabe dadurch gelöst, dass das Verfahren die folgenden Schritte aufweist:
a) Zerkleinern einer Menge an getrockneten Efeublättern,
b) Fermentieren durch Zugabe von Wasser unter Umwandlung von Hederacosid C in α-Hederin,
c) Extrahieren durch Zugabe des Extraktionsmittels, und
d) ggf. Trocknen des Extraktes.

Die der Erfindung zu Grunde liegende Aufgabe wird auf diese Weise vollkommen gelöst.

Mit dem erfindungsgemäßen Verfahren kann nämlich ein Extrakt gewonnen werden, welcher als wirksame Substanz α-Hederin in angereicherter Form aufweist, bzw. im Zuge dessen Herstellung ein Großteil, oder ggf. sämtliches ursprünglich vorliegendes Hederacosid C der Efeublätter in α-Hederin umgewandelt werden kann.

Die Erfinder haben in eigenen Versuchen gezeigt, dass durch den Fermentationsschritt bei getrockneten Efeublättern eine nahezu vollständige Umwandlung von Hederacosid C in α-Hederin gewährleistet wird. Somit sind nach der Extraktion keine weiteren Schritte notwendig, die gewonnenen Inhaltsstoffe weiter umzusetzen. Vielmehr kann gerade durch den zwischengeschalteten Fermentationsschritt das Gesamtverfahren - sowohl im Hinblick auf die Zeitgewinnung, als auch auf die einzusetzenden Materialien - ökonomischer durchgeführt werden, was einen großen Vorteil gegenüber den im Stand der Technik beschriebenen Verfahren darstellt.

"Fermentieren" bedeutet vorliegend der Abbau, bzw. die Umwandlung von in einer Ursubstanz vorliegenden Inhaltsstoffen in andere Stoffe unter Zugabe eines Fermentations-Mediums, bspw. Flüssigkeit, zu der Ursubstanz, wobei ggf. bestimmte Parameter, bspw. Zeit und Temperatur, auf den Fermentations-Prozess abgestimmt werden.

Die Erfinder haben erkannt, dass insbesondere Wasser für den Einsatz beim Fermentationsschritt geeignet ist. Durch die Zugabe von Wasser wird der Umwandlunsprozess von Hederacosid C in α-Hederin angestoßen.

Mit dem erfindungsgemäßen Verfahren kann demnach ein Extrakt mit besonders hohem α-Hederingehalt hergestellt werden kann. Ein Extrakt mit hohem α-Hederinanteil ist insbesondere bei der Behandlung von Erkrankungen der Atemwege wünschenswert, da ja durch die Untersuchungen der Anmelderin festgestellt wurde, dass α-Hederin dafür verantwortlich ist.

Wie oben erwähnt, ist unter den im Efeu enthaltenen Saponinen insbesondere α-Hederin für die spasmolytische Wirkung in den Bronchien verantwortlich. Daher ist dieser Extrakt mit seinem hohen Gehalt an α-Hederin in seiner Wirksamkeit bedeutend verbessert gegenüber Extrakten, in denen noch ein relativ hoher Hederacosid C-Anteil vorliegt.

Getrocknete Drogen haben in der Arzneimittelherstellung den Vorteil, dass sie bezüglich der Haltbarkeit u.U. besser zu handhaben sind als frische Drogen.

Getrocknete Arzneipflanzen und Arzneipflanzenteile werden im Gebiet der Arzneimitteltechnik definitionsgemäß als "Drogen" bezeichnet. Die Verwendung von solchen als "Drogen" vorliegenden Arzneipflanzen erfolgt dabei entweder in unveränderter oder in zerkleinerter Form.

Es ist als für den Fachmann überraschend anzusehen, dass bei getrockneten Efeublättern, wie sie in der pharmazeutischen Industrie eingesetzt werden, eine Umwandlung zu α-Hederin lediglich durch Zugabe von Wasser erzielt werden kann.

Bei der eingangs erwähnten DE 30 25 223 A1 mussten zur Verseifung Alkalihydroxide zugegeben werden, um einen chemischen Vorgang auszulösen.

Weiterhin ist bevorzugt, wenn in Schritt a) eine Zerkleinerung der Efeublätter auf ≤ 5 x 5 mm, und insbesondere auf ≤ 2 x 2 mm, erfolgt.

Eine Zerkleinerung der Efeublätter auf die genannten Maße ist besonders vorteilhaft, um den α-Hederingehalt des erfindungsgemäß hergestellten Extraktes zu beeinflussen. Dabei wurde festgestellt, dass mit zunehmender Teilchengröße insgesamt eine schlechtere Extraktion und Fermentation der Inhaltsstoffe stattfand.

Bei dem erfindungsgemäßen Verfahren ist ferner bevorzugt, wenn das Extraktionsmittel ein Gemisch aus Alkohol- und Wasseranteilen ist.

Insbesondere ist bevorzugt, wenn als Alkohol ein C₂-C₁₀-Alkohol, und insbesondere Ethanol, eingesetzt wird.

Ethanol ist ein bewährtes pharmazeutisches Lösungsmittel und wird im Gebiet der Arzneimitteltechnik als Extraktionsmittel vielfältig eingesetzt. Selbstverständlich können aber auch andere Alkohole, bspw. Propanol, Isopropanol, etc. eingesetzt werden, also alle Alkohole, die als Wasser-/Alkoholgemische bei der Extraktion im Sinne der Arzneimittelzubereitung einsetzbar sind.

Ferner ist bevorzugt, wenn die Wasser-/Alkoholanteile des Extraktionsmittels ein Verhältnis (m/m) von 10/90 bis 90/10 aufweisen, und insbesondere ein Verhältnis von Wasser/Alkohol von 70/30.

Es sind verschiedene Alkohol-/Wasserverhältnisse im Rahmen der vorliegenden Erfindung geeignet, um das erfindungsgemäße Verfahren erfolgreich durchzuführen. Dabei erwies sich der Einsatz eines Extraktionsmittels mit einem Alkoholanteil von 30 % als besonders geeignet.

Ferner ist bevorzugt, wenn das Verhältnis von Efeublätter zu Extraktionsmittel von 1 : 1 bis 1 : 50, und insbesondere von 1 : 12 beträgt.

Durch die Wahl von bestimmten Mengenverhältnissen der zu verarbeitenden Efeublätter und des Extraktionsmittels kann der Anteil von α-Hederin im Extrakt im Zusammenhang mit dem erfindungsgemäßen Verfahren beeinflusst werden. Bei Bestimmung des Mengenverhältnisses muss berücksichtigt werden, dass zum einen Hederacosid C ggf. vollständig umgewandelt werden soll. Andererseits soll die einzusetzende Extraktionsmittelmenge so an das Verfahren angepasst werden, dass bei einem optimal niedrigem Mengeneinsatz eine erfolgreiche Extraktion gewährleistet wird. Dem Fachmann wird klar sein, dass hierzu verschiedene Mengenverhältnisse für einen Einsatz bei dem erfindungsgemäßen Verfahren möglich sind.

In einer Ausführungsform des erfindungsgemäßen Verfahrens ist bevorzugt, wenn in Schritt b) ein Wasseranteil des Extraktionsmittels zugegeben wird, und insbesondere, wenn zunächst lediglich der Wasseranteil von der Hälfte des Extraktionsmittels zugegeben wird.

Bei dieser Ausführungsform erfolgt die Zugabe des Alkoholanteils des Extraktionsmittels, und ggf. die Zugabe von weiterem Extraktionsmittel erst nach dem Fermentieren. Die Zugabe von Alkoholanteilen des Extraktionsmittels nach der Fermentation mit Wasseranteilen ist verfahrenstechnisch von Vorteil, da demnach die einzusetzende Menge an Extraktionsmittel für unterschiedliche Ansätze jeweils vorher exakt und einfach berechnet und eingesetzt werden kann. Die einzelnen Volumenanteile des Wassers und des Alkohols können dann jeweils getrennt voneinander, bzw. einander in den Schritten b) und c) ergänzend, zugegeben werden.

Dabei bietet sich insbesondere an, für das Fermentieren den Wasseranteil von der halben Menge des Extraktionsmittels hinzuzufügen, was das Verfahren noch praktikabler macht. Es ist jedoch nicht ausgeschlossen, dass auch andere Wassermengen zum Zwecke der Fermentation hinzugefügt werden können, um dem Umwandlungsprozess von Hederacosid C in α-Hederin zu beeinflussen.

Ferner ist bevorzugt, wenn Schritt b) für einen Zeitraum von 1 min bis 120 min, vorzugsweise für einen Zeitraum von 60 min, durchgeführt wird.

Durch Variieren des Fermentationszeitraums kann der α-Hederin-Gehalt des Extraktes im Zusammenhang mit dem erfindungsgemäßen Verfahren weiter gesteuert werden.

Dabei ist insbesondere bevorzugt, wenn die Fermentierung bei einer Temperatur zwischen 10 bis 40 °C, insbesondere bei 30 °C, durchgeführt wird.

In einer weiteren Ausführungsform des erfindungsgemäßen Verfahrens ist bevorzugt, wenn das Extrahieren in Schritt c) durch Vorquellen und Perkolation durchgeführt wird.

Beide Maßnahmen sind im Stand der Technik und insbesondere auf dem Gebiet der Arzneimittelzubereitungen aus Pflanzen herkömmliche Methoden. Mit den Schritten des Vorquellens und der Perkolation kann eine erschöpfende Extraktion der Efeublätter erzielt werden. Nach der Fermentation mit Wasseranteilen werden demnach die entsprechenden Alkoholanteile zum Ansatz hinzugefügt, wonach die Vorquellung durchgeführt wird. Zur Perkolation wird vorzugsweise der restliche Anteil des Extraktionsmittels hinzugefügt, von welchem bestimmte Anteile zuvor bei der Fermentation und den anderen Extraktionsschritten eingesetzt wurden.

Hierbei ist bevorzugt, wenn das Vorquellen für einen Zeitraum von 1 h bis 30 h durchgeführt wird, und insbesondere für einen Zeitraum von 6 h.

In einer Weiterführung des erfindungsgemäßen Verfahrens ist bevorzugt, wenn das Trocknen in Schritt d) durch Dünnfilmvaporisation und anschließender Sprühtrocknung durchgeführt wird.

Beide Verfahren sind herkömmliche Trocknungsverfahren, wobei sich die Dünnfilmvaporisation bei der Zubereitung von Arzneimitteln als schonendes Verdampfungsverfahren erwiesen hat, ebenso wie die Sprühtrocknung, mit welcher sich erfahrungsgemäß flüssige Zubereitungen zu pulverförmigen Endprodukten trocknen lassen, die sich insbesondere dadurch auszeichnen, dass sie sich beispielsweise mit Wasser wieder leicht zu gebrauchsfertigen Zubereitungen anrühren lassen.

Ferner ist bevorzugt, wenn das erfindungsgemäße Verfahren zur Steuerung des α-Hederin- und Hederacosid-Gehalts in einem Efeublätterextrakt eingesetzt wird.

Mit dem erfindungsgemäßen Verfahren ist es darüber hinaus möglich, den Anteil an α-Hederin im Extrakt gezielt zu steuern. Danach kann das erfindungsgemäße Verfahren zur vollständigen Umwandlung von Hederacosid in α-Hederin in einem Efeublätterextrakt eingesetzt werden, oder aber nur zur teilweisen Umwandlung von Hederacosid in α-Hederin.

Demnach ist es bspw. möglich, zunächst nur einen bestimmten Teil der Droge der Fermentation mit Wasser zu unterziehen, und nach Ablauf der Fermentation den restlichen Anteil der Droge zur Extraktion hinzuzufügen. Auf diese Weise können gezielt Trockenextrakte hergestellt werden, bei welchen nicht sämtliches Hederacosid C zu α-Hederin umgewandelt wurde.

Ferner ist es dadurch möglich, den Gehalt an α-Hederin im Extrakt konkret zu steuern. Dies ist bspw. dann wünschenswert, wenn der Anteil an α-Hederin im Extrakt nicht so hoch sein soll, sondern vielmehr noch ein bestimmter Anteil an Hederacosid C vorliegen soll.

Die Erfindung betrifft ferner einen Extrakt aus Efeublättern, der nach dem erfindungsgemäßen Verfahren hergestellt ist.

Extrakte mit einem hohen Gehalt an α-Hederin sind bspw. bei der Behandlung von Atemwegserkrankungen von Vorteil, da mit dem hochkonzentriert vorliegenden α-Hederin der aktivste Inhaltsstoff der Blätter hochkonzentriert vorliegt.

Die Erfindung betrifft ferner die Verwendung des erfindungsgemäß hergestellten Extraktes, insbesondere des Trockenextraktes, zur Herstellung eines Arzneimittels, und insbesondere zur Herstellung eines Arzneimittels zur Behandlung von Atemwegserkrankungen.

Weiterhin betrifft die Erfindung ein Arzneimittel, welches einen Extrakt aufweist, der nach dem erfindungsgemäßen Verfahren hergestellt ist.

Dabei kann das Arzneimittel in Form von Kapseln, Tabletten, Dragees, Zäpfchen, Granulat, Pulver, Lösungen, Cremes, Emulsionen, Aerosolen, Salben und Ölen vorliegen. Orale Verabreichungsformen sind dabei insbesondere bevorzugt. Das Arzneimittel kann dabei Hilfsstoffe aufweisen, die herkömmlich in der Arzneimittelherstellung verwendet werden. Eine Reihe von geeigneten Substanzen findet sich bspw. in A. Kibbe, Handbook of Pharmaceutical Excipients, 3. Ed., 2000, American Pharmaceutical Association and pharmaceutical Press.

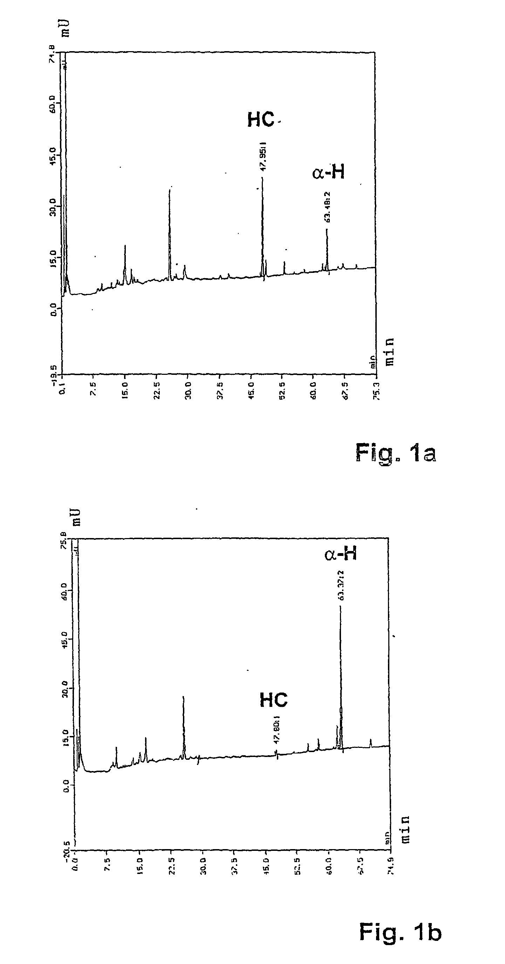

Weitere Vorteile ergeben sich aus der Beschreibung und der beigefügten Figur. Es zeigen
- Fig. 1a: ein Chromatogramm einer Probe, die keiner Fermentation unterzogen wurde, und
- Fig. 1b: ein Chromatogramm einer Probe, die einer Fermentation unterzogen wurde.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

### Beispiel

Das bisher im Stand der Technik durchgeführte Extraktionsverfahren besteht aus einem mehrstufigen Extraktionsverfahren, bei welchem die getrocknete Droge nach dem Schneiden direkt mit 30%igem Ethanol (m/m) versetzt wird. Das Verhältnis von Hederacosid C/α-Hederin des Trockenextraktes entspricht dabei in etwa dem der Droge. Analysen der in den letzten Jahren hergestellten Extrakte zeigen einen Hederacosid C-Gehalt von ca. 5 bis 20 % am Gesamtextrakt, während α-Hederin etwa 1 bis 5 % des Gesamtextraktes ausmacht. Auf Grundlage der im Stand der Technik bekannten Methoden sollte daher ein Verfahren bereitgestellt werden, mit welchem die Umwandlung von Hederacosid C in α-Hederin in das Extraktionsverfahren mit einbezogen werden kann. Hierzu wurden zunächst einige Vorversuche durch geführt:

### 1. Starke und intensive Zerkleinerung der frischen, noch ungetrockneten Blätter mit anschließender Trocknung der Droge

Um den Einfluss des "post harvesting processing" auf die Droge und den späteren Extrakt zu untersuchen, wurden Efeublätter frisch und sorgsam per Hand geerntet. Alle Blätter wurden zur selben Zeit am selben Ort gesammelt und nach ihrer Ernte unterschiedlichen Verfahrensprozessen unterworfen. Anschließend wurden die Proben wie folgt analysiert: Eine Probenmenge, die etwa 200 mg getrockneter Droge entspricht, wurde in der Mühle pulverisiert und zusammen mit 30 ml Ethanol 45 % (v/v) für 1 h geschüttelt. Nach Überführung in ein Chromatographierohr wurde mit frischem Ethanol 45 % (v/v) bis auf ein Volumen von 100 ml perkoliert. Für die Gewinnung unterschiedlicher Proben wurden Variationen bezüglich des Zeitpunktes der Zerkleinerung und der Trocknung durchgeführt:
- Probe 1:: Die frischen Blätter wurden in 3 x 3 mm große Stücke zerschnitten und erst nach 12stündiger Lagerung getrocknet.
- Probe 2:: Die frischen Blätter wurden in 3 x 3 mm große Stücke zerschnitten und anschließend sofort getrocknet.
- Probe 3:: Die ungeschnittenen Blätter wurden nach der Ernte bei 50 °C getrocknet und erst kurz vor dem Extraktionsprozess in ca. 3 x 3 mm große Stücke zerkleinert.

Die nachfolgende Tabelle gibt die chromatographischen Untersuchungen bezüglich des Gehalts an α-Hederin und Hederacosid C wieder:

**Tabelle 1**

| | α-Hederin | Hederacosid C (%) | Gesamtsaponine berechnet als Hederacosid C |
|---|---|---|---|
| Probe 1 | 1,25 | 6,05 | 8,08 |
| Probe 2 | 1,40 | 5,45 | 7,73 |
| Probe 3 | 1,13 | 5,75 | 7,59 |

Die Resultate sind miteinander vergleichbar und weichen nicht signifikant voneinander ab, obwohl man bei den im frischen Zustand geschnittenen Blättern eine geringe Verschiebung des Verhältnisses Hederacosid C/α-Hederin zu Gunsten des α-Hederins zu erkennen scheint.

In weiteren Untersuchungen wurden Variationen bezüglich des Zerkleinerungsgrades der Efeublätter und des Zeitpunktes der Gehaltsbestimmung durchgeführt:
- Probe 1:: Die frischen Blätter wurden fein zerrieben und sofort mit Ethanol 45 % (v/v) extrahiert.
- Probe 2:: Die frischen Blätter wurden fein zerrieben und nach einer Wartezeit von 45 min mit Ethanol 45 % (v/v) extrahiert.
- Probe 3:: Die ungeschnittenen Blätter wurden nach der Ernte bei 50 °C getrocknet und kurz vor dem Extraktionsprozess zerkleinert.

In der nachfolgenden Tabelle 2 sind die Ergebnisse der chromatographischen Untersuchungen bezüglich des α-Hederin- und Hederacosid C-Gehalts dargestellt:

**Tabelle 2**

| | α-Hederin | Hederacosid C (%) | Gesamtsaponine berechnet als Hederacosid C |
|---|---|---|---|
| Probe 1 | 3,74 | 0,37 | 6,45 |
| Probe 2 | 3,17 | 0,19 | 5,34 |
| Probe 3 | 1,13 | 5,75 | 7,59 |

Wie der Tabelle zu entnehmen ist, wurde durch die intensive Zerkleinerung nahezu das komplette Hederacosid C zu α-Hederin umgesetzt.

### 2. Fermentation der getrockneten und zerkleinerten Droge vor der Extraktion

Um die Intaktheit der Zellen zu zerstören, wurde durch starke Zerkleinerung und unter Hinzufügung von Wasser der Umwandlungsprozess von Hederacosid C in α-Hederin angesto-ßen. Dies wurde durch den folgenden Versuch belegt:

Ganze, getrocknete Efeublätter mit einem zuvor bestimmten Gehalt von 7,94 % Hederacosid C und 0,59 % α-Hederin wurden wie folgt behandelt:
- Probe 1:: 200 mg der stark zerkleinerten Blätter werden mit 20 ml Wasser versetzt. Nach Abdampfen des Wassers im Vakuum wird der Ansatz wie üblich mit Ethanol 45 % (v/v) extrahiert.
- Probe 2:: Die Droge wird wie Probe 1 behandelt, aber an Stelle des Wassers wird Ethanol 30 % (m/m) verwendet.

Die chromatographischen Ergebnisse sind bezüglich des Gehaltes an α-Hederin und Hederacosid C in der folgenden Tabelle 3 wiedergegeben:

**Tabelle 3**

| | α-Hederin | Hederacosid C (%) | Gesamtsaponine berechnet als Hederacosid C |
|---|---|---|---|
| Probe 1 | 4,40 | 0 | 7,15 |
| Probe 2 | 1,12 | 6,51 | 8,33 |
| Start | 0,59 | 7,94 | 8,90 |

Wie den Daten zu entnehmen ist, wurde durch Zugabe von Wasser zu der zerkleinerten Droge das Hederacosid C vollständig zu α-Hederin umgebaut.

Fig. 1 zeigt die durch HPLC gewonnenen Chromatogramme der Probe 1 (Fig. 1b) und der Probe 2 (Fig. 1a). In den Figuren ist der für die Substanz Hederacosid C erhaltene Peak mit "HC" bezeichnet, der für α-Hederin erhaltene Peak jeweils mit "α-H". Wie durch den Vergleich der Peaks in den Fig. 1a und 1b zu erkennen ist, wird der Anteil an Hederacosid C auf Kosten des α-Hederin nahezu vollständig reduziert, was zeigt, dass sämtliches Hederacosid C umgewandelt wurde.

Die Fermentation in 30%igem Ethanol (m/m) führte zwar auch zu einem Abbau des Hederacosid C, jedoch in einem deutlich geringeren Ausmaß.

In weiteren Untersuchungen konnte gezeigt werden, dass der Zerkleinerungsgrad der Droge und die zugeführte Menge an Wasser für das Ausmaß der Umwandlung von entscheidender Bedeutung ist: Extraktionsversuche mit ca. 5 x 5 mm großen Blattfragmenten, die wie beschrieben in Wasser fermentiert wurden, zeigten zwar eine Anreicherung des α-Hederins auf Kosten des Hederacosid C, jedoch war die Umsetzung auf Grund der Intaktheit vieler Zellen nicht komplett. Auch der Versuch, fein zerkleinerte Blätter mit Wasser nur leicht zu besprühen, führte zu dem Ergebnis einer unvollständigen Umsetzung des Hederacosid C.

Um die oben genannten bisher gewonnenen Erkenntnisse über die Umwandlung von Hederacosid C in α-Hederin in eine erfolgreiche Extraktionsvorschrift erfolgreich umzusetzen, wurden folgende Varianten eines Extraktionsverfahrens durchgeführt:
- Extrakt 1:: 3,0 g zerkleinerte Efeublätter (5 x 5 mm) wurden mit der 12fachen Menge an Ethanol 30 % (m/m) extrahiert (normale Extraktion).
- Extrakt 2:: 3,0 g zerkleinerte Efeublätter (5 x 5 mm) wurden in einer Chromatographiesäule mit Wasser bedeckt. Nach 1 h wurde das Wasser über einen Auslasshahn abgelassen, um anschließend mit der 12fachen Menge Ethanol 30 % (m/m) zu extrahieren.
- Extrakt 3:: Zu 3,0 g zerkleinerten Efeublättern (5 x 5 mm) wurde das Wasser aus 12 Teilen Extraktionsmittel (Ethanol 30 % (m/m)) zugesetzt. Nach 1 h Fermentationszeit wurde der Ethanolanteil aus 12 Teilen Extraktionsmittel zugesetzt, um zu extrahieren.

Die chromatographischen Auswertungen ergaben die in der folgenden Tabelle 4 gezeigten Ergebnisse:

**Tabelle 4**

| | α-Hederin | Hederacosid C (%) | Gesamtsaponine berechnet als Hederacosid C |
|---|---|---|---|
| Extrakt 1 | 0,48 | 19,80 | 20,58 |
| Extrakt 2 | 4,11 | 1,38 | 8,06 |
| Extrakt 3 | 8,38 | 5,88 | 19,51 |

Da die Droge bei diesen Extraktionen nur auf 5 x 5 mm zerkleinert wurde, erfolgte die Umwandlung nicht im kompletten Umfang. Aus der Tabelle ist deutlich zu erkennen, dass das Ablassen des Fermentationswassers (Extrakt 2) zu Verlusten der Inhaltsstoffe führte.

Unter Kombination der oben geschilderten Vorversuche wurde ein weiteres Extraktionsverfahren entwickelt, mit welchem der α-Hederin-Gehalt eines Efeublättertrockenextraktes gesteuert werden kann.

### 3. Extraktionsverfahren zur Herstellung von Efeublättertrockenextrakten mit steuerbarem α-Hederin-Gehalt

Nach Prüfung der Qualität und Freigabe durch die Qualitätskontrolle wurde ein Teil der Droge (Efeublätter DAC) in einer Mühle stark zerkleinert, wobei eine Schutzsiebung eine Größe der Fragmente von maximal 2 x 2 mm garantierte. Zusätzlich wurde das gesiebte Gut optisch auf größere Partikel und Verunreinigungen geprüft.

Zu der zerkleinerten Probe wurde der Wasseranteil aus 6 Teilen Extraktionsmittel (Ethanol 30 % (m/m)) gegeben. Dieser Ansatz wurde bei 30 °C unter gelegentlichem Vermischen/Umrühren für 60 min fermentiert.

Anschließend wurde der Anteil Ethanol 96 % aus 6 Teilen des Extraktionsmittels zugegeben und der Ansatz durch Rühren homogenisiert.

Nach einer 6-stündigen Vorquellphase wurde das Eluat abgetrennt und die zurückbleibende Droge mit den restlichen 6 Teilen des Extraktionsmittels perkoliert.

Die vereinten Eluate wurden zum Ausschluss kleiner Drogenpartikel nochmals filtriert und homogenisiert, bevor sie mittels Dünnfilmvaporisation bei 55 °C und 150 mbar zum Spissum getrocknet wurden. Dieses wurde homogenisiert und anschließend per Sprühtrocknung bei 45 bis 60 °C zum Efeublättertrockenextrakt getrocknet.

Zur Überprüfung dieses Herstellungsverfahrens wurden folgende Extrakte hergestellt: Ausgehend von Efeublättern mit einem Gehalt von 3,91 % Hederacosid C und 0,20 % α-Hederin wurde einmal nach der konventionellen Methode (Zerkleinern der getrockneten Probe mit direkt anschließendem Versetzen mit 30% (m/m) Ethanol und Extraktion) und einmal nach der neuen Methode ein Extrakt angefertigt. In der nachfolgenden Tabelle 5 sind die chromatographischen Ergebnisse bezüglich des Gehaltes an α-Hederin und Hederacosid wiedergegeben:

**Tabelle 5**

| | α-Hederin (%) | Hederacosid C (%) | Gesamtsaponine berechnet als Hederacosid C (%) |
|---|---|---|---|
| Konventionell | 0,53 | 8,68 | 9,54 |
| Neu | 4,74 | 0 | 7,71 |

Wie der Tabelle zu entnehmen ist, konnte mit dem neuen Extraktionsverfahren das in den Blättern enthaltene Hederacosid C vollständig in α-Hederin umgewandelt werden.

Da sich auch der Gesamtsaponingehalt, berechnet als Hederacosid C, in derselben Größenordnung bewegt, ist man - bei Kenntnis der entsprechenden Saponinkonzentration in der Droge und unter Berücksichtigung des Anreicherungsfaktors von ca. 2 bis 3 - in der Lage, den letztendlichen α-Hederin-Gehalt des Extraktes abschätzen.

Um nur einen Teil des in der Droge vorliegenden Hederacosid C in α-Hederin umzuwandeln, kann demnach nur ein bestimmter Anteil der Droge der Fermentation mit Wasser unterzogen werden, wobei alle anderen Parameter konstant bleiben. Nach Ablauf der 60-minütigen Fermentation wird dann für die 6-stündige Vorquellung die restliche Droge und das Ethanol zugesetzt.

Mit dem erfindungsgemäßen Verfahren ist es demnach möglich, das Inhaltsstoffspektrum eines Efeublättertrockenextraktes durch Einführung einer Fermentation deutlich zu ändern, ohne einen großen Zeit- und Ressourcenaufwand anzuwenden. Nicht zuletzt durch die zahlreichen Veröffentlichungen über die Wirksamkeit von α-Hederin wirkt sich die gezielte Beeinflussung des α-Hederin-Gehalts in einem Trockenextrakt, bzw. Arzneimittels, auf die Wirksamkeit des Extraktes positiv aus.

## Patentansprüche

1. Verfahren zur Herstellung eines Extraktes aus Efeublättern unter Verwendung eines Extraktionsmittels, wobei der Extrakt α-Hederin aufweist, **dadurch gekennzeichnet, dass** es die folgenden Schritte aufweist:
a) Zerkleinern einer Menge an getrockneten Efeublättern,
b) Fermentieren durch Zugabe von Wasser unter Umwandlung von Hederacosid C in α-Hederin;
c) Extrahieren durch Zugabe des Extraktionsmittels, und
d) ggf. Trocknen des Extraktes.

2. Verfahren nach Anspruch 1, **dadurch gekennzeichnet, dass** in Schritt a) eine Zerkleinerung der Efeublätter auf ≤ 5 x 5 mm erfolgt.

3. Verfahren nach Anspruch 2, **dadurch gekennzeichnet, dass** die Zerkleinerung der Efeublätter auf ≤ 2 x 2 mm erfolgt.

4. Verfahren nach einem der Ansprüche 1 bis 3, **dadurch gekennzeichnet, dass** als Extraktionsmittel ein Gemisch aus Alkohol- und Wasseranteilen eingesetzt wird.

5. Verfahren nach einem der Ansprüche 1 bis 4, **dadurch gekennzeichnet, dass** im Extraktionsmittel als Alkohol ein C₂-C₁₀-Alkohol eingesetzt wird.

6. Verfahren nach Anspruch 5, **dadurch gekennzeichnet, dass** als Alkohol 96%iges Ethanol eingesetzt wird.

7. Verfahren nach einem der Ansprüche 1 bis 6, **dadurch gekennzeichnet, dass** die Wasser-/Alkoholanteile des Extraktionsmittels ein Verhältnis (m/m) von 10/90 bis 90/10 betragen.

8. Verfahren nach Anspruch 7, **dadurch gekennzeichnet, dass** das Verhältnis Wasser/Alkohol 70/30 beträgt.

9. Verfahren nach einem der Ansprüche 1 bis 8, **dadurch gekennzeichnet, dass** das Verhältnis von Efeublätter zu Extraktionsmittel von 1 : 1 bis 1 : 50 beträgt.

10. Verfahren nach Anspruch 9, **dadurch gekennzeichnet, dass** das Verhältnis von Efeublätter zu Extraktionsmittel 1 : 12 beträgt.

11. Verfahren nach einem der Ansprüche 1 bis 9, **dadurch gekennzeichnet, dass** in Schritt b) ein Wasseranteil des Extraktionsmittels eingesetzt wird.

12. Verfahren nach Anspruch 11, **dadurch gekennzeichnet, dass** in Schritt b) der Wasseranteil von der Hälfte der Gesamtmenge an Extraktionsmittel zugegeben wird.

13. Verfahren nach einem der Ansprüche 10 bis 12, **dadurch gekennzeichnet, dass** der Wasseranteil von 6 Teilen des Extraktionsmittel zugegeben wird.

14. Verfahren nach einem der Ansprüche 1 bis 13, **dadurch gekennzeichnet, dass** Schritt b) für einen Zeitraum von 1 min bis 120 min durchgeführt wird.

15. Verfahren nach Anspruch 14, **dadurch gekennzeichnet, dass** Schritt b) für einen Zeitraum von 60 min durchgeführt wird.

16. Verfahren nach einem der Ansprüche 1 bis 15, **dadurch gekennzeichnet, dass** Schritt b) bei einer Temperatur von 10 bis 40 °C durchgeführt wird.

17. Verfahren nach Anspruch 16, **dadurch gekennzeichnet, dass** Schritt b) bei einer Temperatur von 30 °C durchgeführt wird.

18. Verfahren nach einem der Ansprüche 1 bis 17, **dadurch gekennzeichnet, dass** das Extrahieren in Schritt c) durch Vorquellen und Perkolation durchgeführt wird.

19. Verfahren nach Anspruch 18, **dadurch gekennzeichnet, dass** das Vorquellen für einen Zeitraum von 1 h bis 30 h durchgeführt wird.

20. Verfahren nach Anspruch 19, **dadurch gekennzeichnet, dass** das Vorquellen für einen Zeitraum von 6 h durchgeführt wird.

21. Verfahren nach einem der Ansprüche 1 bis 20, **dadurch gekennzeichnet, dass** das Trocknen in Schritt d) durch Dünnfilmvaporisation und anschließender Sprühtrocknung durchgeführt wird.

22. Verfahren nach einem der Ansprüche 1 bis 21 zur Steuerung des α-Hederin- und Hederacosid-Gehalts in einem Efeublätterextrakt.

23. Extrakt aus Efeublättern, **dadurch gekennzeichnet, dass** er gemäß dem Verfahren nach einem der Ansprüche 1 bis 22 hergestellt ist.

24. Verwendung eines Extraktes nach Anspruch 23 zur Herstellung eines Arzneimittels.

25. Verwendung nach Anspruch 24, **dadurch gekennzeichnet, dass** das Arzneimittel zur Behandlung von Atemwegserkrankungen eingesetzt wird.

26. Arzneimittel, welches einen Extrakt nach Anspruch 25 aufweist.

## Claims

1. Process for preparing an extract of ivy leaves using an extracting agent, with the extract comprising α-hederin, **characterized in that** it comprises the following steps:
a) comminuting a quantity of dried ivy leaves,
b) fermenting by adding water, with hederacoside C being converted into α-hederin,
c) extracting by adding the extracting agent, and
d) where appropriate, drying the extract.

2. Process of claim 1, **characterized in that** the ivy leaves are comminuted in step a) to ≤ 5 × 5 mm.

3. Process of claim 2, **characterized in that** the ivy leaves are comminuted to ≤ 2 × 2 mm.

4. Process of anyone of claims 1 to 3, **characterized in that** a mixture of alcohol and water fractions is employed as the extracting agent.

5. Process of anyone of claims 1 to 4, **characterized in that** the alcohol employed in the extracting agent is a C₂-C₁₀-alcohol.

6. Process of claim 5, **characterized in that** the alcohol employed is 96% ethanol.

7. Process of anyone of claims 1 to 6, **characterized in that** the water/alcohol fractions in the extracting agent are in a ratio (m/m) of from 10/90 to 90/10.

8. Process of claim 7, **characterized in that** the water/alcohol ratio is 70/30.

9. Process of one of anyone of claims 1 to 8, **characterized in that** the ratio of ivy leaves to extracting agent is from 1:1 to 1:50.

10. Process of claim 9, **characterized in that** the ratio of ivy leaves to extracting agent is 1:12.

11. Process of anyone of claims 1 to 9, **characterized in that** a water fraction of the extracting agent is employed in step b).

12. Process of claim 11, **characterized in that** the water fraction from half of the total quantity of extracting agent is added in step b).

13. Process of anyone of claims 10 to 12, **characterized in that** the water fraction from 6 parts of the extracting agent is added.

14. Process of anyone of claims 1 to 13, **characterized in that** step b) is carried out for a period of from 1 min to 120 min.

15. Process of claim 14, **characterized in that** step b) is carried out for a period of 60 min.

16. Process of anyone of claims 1 to 15, **characterized in that** step b) is carried out at a temperature of from 10 to 40°C.

17. Process of claim 16, **characterized in that** step b) is carried out at a temperature of 30°C.

18. Process of anyone of claims 1 to 17, **characterized in that** the extraction in step c) is carried out by preswelling and percolation.

19. Process of claim 18, **characterized in that** the preswelling is carried out for a period of from 1 h to 30 h.

20. Process of claim 19, **characterized in that** the preswelling is carried out for a period of 6 h.

21. Process of anyone of claims 1 to 20, **characterized in that** the drying in step d) is carried out by thin film vaporization and subsequent spray drying.

22. Process of anyone of claims 1 to 21 for controlling the contents of α-hederin and hederacoside in an ivy leaf extract.

23. Extract of ivy leaves, **characterized in that** it is produced using the process as claimed in one of claims 1 to 22.

24. Use of an extract as claimed in claim 23 for preparing a pharmaceutical.

25. Use of claim 24, **characterized in that** the pharmaceutical is employed for treating respiratory tract diseases.

26. Pharmaceutical which comprises an extract as claimed in claim 25.

## Revendications

1. Procédé de fabrication d'un extrait de feuilles de lierre recourant à un moyen d'extraction, l'extrait contenant de l'α-hédérine, **caractérisé en ce qu'**il comporte les étapes suivantes :
a) broyage d'une quantité de feuilles de lierre séchées,
b) fermentation par ajout d'eau, l'hédéracoside C étant convertie en α-hédérine,
c) extraction par ajout du moyen d'extraction, et
d) séchage de l'extrait le cas échéant.

2. Procédé selon la revendication 1, **caractérisé en ce qu'**un broyage des feuilles de lierre à une dimension ≤ 5 x 5 mm est effectué lors de l'étape a).

3. Procédé selon la revendication 2, **caractérisé en ce que** les feuilles de lierre sont broyées à une dimension ≤ 2 x 2 mm.

4. Procédé selon l'une des revendications 1 à 3, **caractérisé en ce qu'**un mélange de parts d'alcool et d'eau est mis en oeuvre comme moyen d'extraction.

5. Procédé selon l'une des revendications 1 à 4, **caractérisé en ce qu'**un alcool C₂-C₁₀ est mis en oeuvre en tant qu'alcool dans le moyen d'extraction.

6. Procédé selon la revendication 5, **caractérisé en ce que** de l'éthanol à 96 % est mis en oeuvre en tant qu'alcool.

7. Procédé selon l'une des revendications 1 à 6, **caractérisé en ce que** le rapport (m/m) entre les parts eau/alcool du moyen d'extraction est compris entre 10/90 et 90/10.

8. Procédé selon la revendication 7, **caractérisé en ce que** le rapport eau/alcool est de 70/30.

9. Procédé selon l'une des revendications 1 à 8, **caractérisé en ce que** le rapport entre les feuilles de lierre et le moyen d'extraction est compris entre 1 : 1 et 1 : 50.

10. Procédé selon la revendication 9, **caractérisé en ce que** le rapport entre les feuilles de lierre et le moyen d'extraction est de 1 : 12.

11. Procédé selon l'une des revendications 1 à 9, **caractérisé en ce qu'**une part d'eau du moyen d'extraction est mise en oeuvre lors de l'étape b).

12. Procédé selon la revendication 11, **caractérisé en ce que** la part d'eau de la moitié de la quantité totale de moyen d'extraction est ajoutée lors de l'étape b).

13. Procédé selon l'une des revendications 10 à 12, **caractérisé en ce que** la part d'eau de 6 fractions du moyen d'extraction est ajoutée.

14. Procédé selon l'une des revendications 1 à 13, **caractérisé en ce que** l'étape b) est exécutée pendant une durée comprise entre 1 minute et 120 minutes.

15. Procédé selon la revendication 14, **caractérisé en ce que** l'étape b) est exécutée pendant une durée de 60 minutes.

16. Procédé selon l'une des revendications 1 à 15, **caractérisé en ce que** l'étape b) est exécutée à une température comprise entre 10 et 40° C.

17. Procédé selon la revendication 16, **caractérisé en ce que** l'étape b) est exécutée à une température de 30°C.

18. Procédé selon l'une des revendications 1 à 17, **caractérisé en ce que** l'extraction est effectuée par macération et percolation lors de l'étape c).

19. Procédé selon la revendication 18, **caractérisé en ce que** la macération est effectuée pendant une durée comprise entre 1 heure et 30 heures.

20. Procédé selon la revendication 19, **caractérisé en ce que** la macération est effectuée pendant une durée de 6 heures.

21. Procédé selon l'une des revendications 1 à 20, **caractérisé en ce que** le séchage est effectué par vaporisation d'un film mince et séchage par atomisation consécutif lors de l'étape d).

22. Procédé selon l'une des revendications 1 à 21 pour le contrôle de la teneur en α-hédérine et en hédéracoside d'un extrait de feuilles de lierre.

23. Extrait de feuilles de lierre, **caractérisé en ce qu'**il est fabriqué conformément au procédé selon l'une des revendications 1 à 22.

24. Utilisation d'un extrait selon la revendication 23 pour la fabrication d'un médicament.

25. Utilisation selon la revendication 24, **caractérisée en ce que** le médicament est mis en oeuvre pour le traitement d'affections des voies respiratoires.

26. Médicament contenant un extrait selon la revendication 25.
